# EUROPÄISCHE PATENTSCHRIFT

(11) **EP 1 053 997 B1**
(45) Veröffentlichungstag und Bekanntmachung des Hinweises auf die Patenterteilung: **28.02.2007**
(21) Anmeldenummer: 00109145.3
(22) Anmeldetag: 05.05.2000
(51) Int. Cl.: C07C 253/30, C07C 255/09, C07F 15/00

(54) **Herstellung von geradkettigen Acrylnitril-Dimeren**
Process for the preparation of linear acrylonitril dimers
Procédé pour la préparation de dimères linéaires d'acrylonitrile

(30) Priorität: 18.05.1999 DE 19922642
(43) Veröffentlichungstag der Anmeldung: 22.11.2000
(73) Patentinhaber: Saltigo GmbH, 51369 Leverkusen (DE)
(72) Erfinder: Gürtler, Christoph, Dr., 50676 Köln (DE); Braun, Gerhard, Dr., 50678 Köln (DE); Jautelat, Manfred, Dr., 51399 Burscheid (DE)
(74) Vertreter: Wichmann, Birgid

(56) Entgegenhaltungen:
- US-A- 5 466 858
- BUDAVARI S (ED): "The Merck Index 12th Edition" , MERCK INDEX. ENCYCLOPEDIA OF CHEMICALS, DRUGS, AND BIOLOGICALS. 12TH. EDITION 1996, WHITEHOUSE STATION, MERCK & CO, US, VOL. ED. 12, PAGE(S) 1044 XP002147533 ISBN: 0-911910-12-3 * Absatz [6200] *
- "Alphabetical list of compounds. Hydroxy" , SIGMA CATALOGUE, XX, XX, PAGE(S) 557 XP002147534 * Seite 557, Absatz H5501 *

## Beschreibung

Die vorliegende Erfindung betrifft ein Verfahren zur Herstellung von geradkettigen Acrylnitril-Dimeren aus Acrylnitril in Gegenwart von Additiven.

Geradkettige Acrylnitril-Dimere sind als Zwischenprodukte für die Herstellung von Rostschutzmitteln, Vulkanisationsverfahren und Kautschukmaterialen von Bedeutung. Ein weiterer wichtiger Einsatzbereich für geradkettige Acrylnitril-Dimere ist die Herstellung von Hexamethylendiamin, welches für die Herstellung von Nylon wesentliche Bedeutung hat.

Die Dimerisierung von Acrylnitril in Gegenwart von Rutheniumkatalysatoren und Wasserstoff ist in D.T. Tsou et al., *J. Mol. Catal.* 22(1). 1983, 29-45 beschrieben.

Die Anwesenheit von Wasserstoff führt jedoch dazu, dass als Nebenreaktion die Hydrierung von Acrylnitril zu Propionitril abläuft, welches in größeren Mengen (33 bis 45% Ausbeute) entsteht.

Die Dimerisierung von Acrylnitril in Gegenwart von Rutheniumkatalysatoren ohne Wasserstoffatmosphäre ist in DE-A-44 31 307 beschrieben.

Bei der Dimerisierung werden als Additive Carbonsäuren eingesetzt, wodurch die Propionitrilbildung zurückgedrängt wird. Carbonsäuren haben jedoch den Nachteil, dass sie mit Acrylnitril zu β-Cyanoestern reagieren. Dadurch sinkt die theoretisch erreichbare Ausbeute an Acrylnitril-Dimeren. Darüberhinaus ist die Entfernung von β-Cyanoestern aus dem Produktgemisch nur mit zeitaufwendigen Prozeduren durchführbar. Dabei müssen die β-Cyanoester erst durch eine thermische Spaltung in die Ausgangskomponenten überführt werden bevor diese dann destillativ von den Produkten abgetrennt werden können.

Aufgabe der vorliegenden Erfindung war es nun, ein Verfahren für die Dimerisierung von Acrylnitril bereitzustellen, bei dem die β-Cyanoesterbildung unterdrückt und die Bildung von geradkettigen Acrylnitril-Dimeren mit hoher Selektivität ermöglicht wird.

Es wurde nun ein Verfahren zur Herstellung von geradkettigen Acrylnitril-Dimeren gefunden, das dadurch gekennzeichnet ist, dass Acrylnitril in Gegenwart eines Rutheniumkatalysators und zusätzlich in Gegenwart eines Additivs dimerisiert wird, wobei diese Additive
aromatische Hydroxyverbindungen sind, die mindestens einen Alkyloxycarbonyl-Substituenten und mindestens einen weiteren Substituenten R am aromatischen Grundkörper tragen
oder
heteroaromatische Hydroxyverbindungen sind, die mindestens einen Alkyloxycarbonyl-Substituenten und mindestens einen weiteren Substituenten R am heteroaromatischen Grundkörper tragen,
wobei R unabhängig voneinander
Halogen, Cyano, Amino, Amido, Urethan, Sulfonyl, Phosphonyl, Formyl, geradkettiges oder verzweigtes C₁-C₁₀-Alkyl, C₁-C₁₀-Alkoxy, C₁-C₁₀-Alkyloxo, C₁-C₁₀-Alkylsulfinyl, C₁-C₁₀-Alkylamino, C₁-C₁₀-Halogenalkyl oder C₁-C₁₀- Alkyloxycarbonyl bedeutet
und
die heteroaromatische Hydroxyverbindung als Heteroatom Stickstoff, Schwefel und/oder Sauerstoff enthält.

Die Additive verstärken zum einen die Selektivität für geradkettige Acrylnitril-Dimere und unterdrücken zum anderen die β-Cyanoesterbildung vollständig.

Als aromatische Hydroxyverbindungen werden bevorzugt Phenolderivate, Hydroxynaphthalinderivate oder Hydroxyanthracenderivate eingesetzt, besonders bevorzugt werden Phenolderivate eingesetzt.

Werden als Additive Phenolderivate eingesetzt, steht der Alkyloxycarbonyl-Substituent und/oder der Substituent R bezüglich der phenolischen Gruppe bevorzugt in ortho-und/oder para-Stellung.

Als heteroaromatische Hydroxyverbindungen werden bevorzugt Hydroxythiophenderivate, Hydroxyfuranderivate, Hydroxypyrrolderivate, Hydroxypyridinderivate oder Hydroxyimidazolderivate eingesetzt.

In einer bevorzugten Ausführungsform handelt es sich bei dem Alkyloxycarbonyl-Substituenten um geradkettiges oder verzweigtes C₁-C₁₀- Alkyloxycarbonyl, besonders bevorzugt um Methyloxycarbonyl.

Bei den Substituenten R handelt es sich bevorzugt um Formyl, geradkettiges oder verzweigtes C₁-C₁₀-Alkyloxo, geradkettiges oder verzweigtes C₁-C₁₀- Alkyloxycarbonyl oder geradkettiges oder verzweigtes C₁-C₁₀-Alkyl, ganz besonders bevorzugte Substituenten R sind Methyloxycarbonyl, Methyl, Acetyl oder Hydroxyl.

Ganz besonders bevorzugte Additive sind 2-Hydroxy-1,3,5-benzoltricarbonsäuretrimethyl-ester, 2,4-Dihydroxy-3,6-dimethylbenzoesäuremethylester, 5-Acetyl-2-hydroxy-benzoesäure-methylester oder 2-Hydroxy-1,5-benzoldicarbonsäuredimethylester.

In einer bevorzugten Ausführungsform des erfindungsgemäßen Verfahrens werden das Additiv und das Acrylnitril in einem molaren Verhältnis von (0,001 bis 5):1, besonders bevorzugt (0,005 bis 2):1, ganz besonders bevorzugt (0,01 bis 0,1):1 eingesetzt.

In einer bevorzugten Ausführungsform des erfindungsgemäßen Verfahrens werden als Rutheniumkatalysatoren Rutheniumsalze von anorganischen oder organischen Säuren oder Rutheniumkomplexe eingesetzt, besonders bevorzugt werden Rutheniumkomplexe eingesetzt.

Bevorzugte Rutheniumsalze von anorganischen Säuren sind: Rutheniumchlorid, Rutheniumbromid, Rutheniumiodid, Rutheniumnitrat oder Rutheniumsulfat.

Bevorzugte Rutheniumsalze von organischen Säuren sind Rutheniumacetat, Rutheniumpropionat, Rutheniumbutanoat, Rutheniumpentanoat, Ruthenium-hexanoat, Rutheniumstearat, Rutheniumnaphthenat, Rutheniumoxalat oder Rutheniumsuccinat.

Bevorzugte Rutheniumkomplexe sind Dichloro-tetraacrylnitril-ruthenium, Dichloro-tris-(triphenylphosphin)-ruthenium, Dichloro-tetrakis-(triphenylphosphin)-ruthenium, Tris-(dimethylsulfoxid)-ruthenium, Dichloro-tetrakis-(dimethylsulfoxid)ruthenium, Dichloro-tetrakis(diphenylsulfoxid)-ruthenium, Dichloro-tetrakis(diphenylsulfid)-ruthenium, Dibromo-tetrakis-(dimethylsulfoxid)-ruthenium, Dichloro-cyclooctadien-ruthenium, Dichloro-1,4-dicyanobutadien-ruthenium, Dichloro-bis-(acetylendicarbon-säuremethylester)-ruthenium, Ruthenium-bis-acetylacetonat, Dichloro-tris-(triphenoxyphosphin)-ruthenium oder Dichloro-tetrakis-(dipropylsulfoxid)-ruthenium

Besonders bevorzugte Rutheniumkomplexe sind Dichloro-tetrakis-(dimethylsulfoxid)-ruthenium, Dichloro-tetrakis-(diphenylsulfoxid)-ruthenium, Dichloro-tetrakis-(diphenylsulfid)-ruthenium, Dibromo-tetrakis-(dimethylsulfoxid)-ruthenium, Dichloro-cyclooctadien-ruthenium, Dichloro-1,4-dicyanobutadien-ruthenium, Dichloro-bis-(acetylendicarbonsäure-methylester)-ruthenium, Ruthenium-bis-acetylacetonat, Dichloro-tris-(triphenoxy-phosphin)-ruthenium oder Dichloro-tetrakis-(dipropylsulfoxid)-ruthenium.

Ganz besonders bevorzugt wird als Rutheniumkatalysator Dichloro-tetrakis-(diphenylsulfoxid)-ruthenium eingesetzt, welches durch Umsetzung von Dichloro-cyclooctadien-ruthenium mit Diphenylsulfoxid in Toluol erhältlich ist.

Der für das erfindungsgemäße Verfahren verwendete Rutheniumkatalysator wird in einer Menge von 0,0001 bis 5 mol%, bezogen auf die Menge an eingesetztem Acrylnitril, eingesetzt. Bevorzugt wird der Katalysator in einer Menge von 0,001 bis 2,5 mol% eingesetzt, besonders bevorzugt 0,05 bis 0, 1 mol%.

Die durch das erfindungsgemäße Verfahren bevorzugt erhaltenen linearen Acrylnitril-Dimere sind 1,4 Dicyanobuten, 1,4 Dicyanobutadien und Adiponitril.

Das erfindungsgemäße Verfahren erlaubt die Dimerisierung von Acrylnitril in einem Reaktionsmedium oder ohne die Verwendung eines Reaktionsmediums. Bevorzugt wird die Dimerisierung in einem Reaktionsmedium durchgeführt.

Für das erfindungsgemäße Verfahren kann das Reaktionsmedium Nitrile, Sulfoxide, Ether, Kohlenwasserstoffe, halogenierte Kohlenwasserstoffe, Amide, Ester, ionischen Flüssigkeiten oder Wasser enthalten, wobei auch Mischungen dieser Verbindungen eingesetzt werden können.

Bevorzugt wird die Dimerisierung in einem Reaktionsmedium durchgeführt wird, welches mindestens eine Verbindung, wählbar aus Nitrilen, Sulfoxiden, Ether, Kohlenwasserstoffen, halogenierten Kohlenwasserstoffen, Amiden, Estern oder Wasser enthält.

Besonders bevorzugt wird die Dimerisierung in Acetonitril als Reaktionsmedium durchgeführt.

Das erfindungsgemäße Verfahren kann in einem Temperaturbereich von 70°C bis 220°C durchgeführt werden. Bevorzugt wird in einem Temperaturbereich von 100°C bis 200°C, ganz besonders bevorzugt in einem Temperaturbereich von 110°C bis 180°C gearbeitet.

Üblicherweise liegt der Reaktionsdruck für das erfindungsgemäße Verfahren in einem Bereich von 0,1 bar bis 50 bar. Bevorzugt wird bei 1 bar bis 30 bar gearbeitet, besonders bevorzugt bei 5 bar bis 25 bar.

In einer bevorzugten Ausführungsform des erfindungsgemäßen Verfahrens wird Acrylnitril in Gegenwart von Dichloro-tetrakis-(diphenylsulfoxid)-ruthenium und in Gegenwart des Additivs 2-Hydroxy-1,5-benzoldicarbonsäuredimethylester in Acetonitril als Reaktionsmedium dimerisiert.

### Beispiele

### Beispiele 1 bis 11

Die Beispiele wurden in einem 30 ml Autoklaven ohne Wandungen unter magnetischer Rührung durchgeführt. Die in der nachfolgenden Tabelle 1 aufgeführten Katalysatoren 1 bis 11 (0,057 mmol; 0,1 mol% bezogen auf Acrylnitril) wurde zusammen mit dem Additiv 2-Hydroxy-1,5-benzoldicarbonsäuredimethylester (479 mg, 2,3 mmol; 4 mol% bezogen auf Acrylnitril), Acrylnitril (3,72 ml; 57 mmol) und 10 ml Acetonitril vorgelegt. Der Autoklav wurde zweifach evakuiert und anschließend mit Stickstoff geflutet. Die Dimerisierung wurde bei einem Stickstoffdruck von 20 bar durchgeführt. Beispiel 8 dient als Vergleichsbeispiel.

**Tabelle 1**

| Beispiel | Katalysator | Reaktionszeit (h) | Ausbeute an linearen Dimeren (%) | Selektivität bezüglich linearen Dimeren (%) |
|---|---|---|---|---|
| 1 | RuCl₂COD | 12 | 9 | 82 |
| 2 | RuCl₂(DMSO)₄ | 12 | 12 | 82 |
| 3 | RuCl₂(Acetylendicarbonsäure-dimethylester)₂ | 3 | 4 | 72 |
| 4 | RuCl₂(1,4 Dicyanobutadien) | 3 | 2 | 68 |
| 5 | RuCl₂(DPhSO)₄ | 18 | 13 | 87 |
| 6 | Ru(acac)₂ | 3 | 5 | 5 |
| 7 | RuCl₂[P(OPh₃)]₃ | 12 | 4 | 87 |
| 8 | OsCl₂(DMSO)₄ | | 0 | - |
| 9 | RuBr₂(DMSO)₄ | 12 | 4 | 73 |
| 10 | RuCl₂(DPrSO)₄ | 12 | 6 | 79 |
| 11 | RuCl₂(Diphenylsulfid)₄ | 12 | 10 | 81 |

| | | | | |
|---|---|---|---|---|
| COD = Cyclooctadien, DMSO = Dimethylsulfoxid, DPhSO = Diphenylsulfoxid, acac = Acetylacetonat, Ph = Phenyl, Pr = Propyl. | | | | |

### Herstellung von Dichloro-tetrakis-(diphenylsulfoxid)-ruthenium

In einem 10 ml Schlenkrohr wurden 3,5 ml entgastes Toluol vorgelegt und 292 mg RuCl₂COD darin gelöst. Zur Lösung wurden 805 mg Diphenylsulfoxid zugegeben und die Mischung wurde 1 h bei 100° C gerührt. Nach Beendigung der Reaktion wurde die Mischung langsam abgekühlt. Zur Kristallisation des Reaktionsprodukts wurde die Reaktionslösung unter Argon bei 4° C 12 h lang stehen gelassen. Der entstandene Niederschlag wurde abgesaugt, mit Petrolether gewaschen und im Hochvakuum getrocknet. Die Ausbeute betrug 28% der Theorie.

### Beispiel 12 bis 20

Die Beispiele wurden in einem 30 ml Autoklaven ohne Wandungen unter magnetischer Rührung durchgeführt. Der Katalysator Dichloro-tetrakis-(diphenylsulfoxid)-ruthenium (56 mg; 0,057 mmol; 0,1 mol% bezogen auf Acrylnitril) wurde zusammen mit den in Tabelle 2 angegebenen Additiven 12-20 (4 mol% bezogen auf Acrylnitril), Acrylnitril (3,72 ml; 57 mmol) und 10 ml Acetonitril vorgelegt. Der Autoklav wurde zweifach evakuiert und anschließend mit Stickstoff geflutet. Die Dimerisierung wurde bei einem Stickstoffdruck von 20 bar durchgeführt.

Die Beispiele 12 bis 16 dienen als Vergleichsversuche.

**Tabelle 2**

| Beispiel | Additiv | Reaktionszeit (h) | Ausbeute an linearen Dimeren (%) | Selektivität bezüglich linearen Dimeren (%) |
|---|---|---|---|---|
| 12 | CH₃(CH₂)₂PO₄H(CH₂)₂CH₃ | 12 | 0 | - |
| 13 | 2-Hydroxybenzonitril | 3 | 0 | - |
| 14 | 2-Acetamidophenol | 3 | 0 | - |
| 15 | 2-Hydroxybenzoesäure-methylester | 12 | 9 | 55 |
| 16 | 4-Hydroxybenzoesäure-methylester | 12 | 8 | 50 |
| 17 | 2-Hydroxy-1,3,5-benzoltricarbonsäuretrimethylester | 12 | 8 | 42 |
| 18 | 2,4-Dihydroxy-3,6-dimethylbenzoesäuremethylester | 12 | 5 | 69 |
| 19 | 5-Acetyl-2-hydroxybenzoesäuremethylester | 3 | 8 | 78 |
| 20 | 2-Hydroxy-1,5-benzoldicarbonsäuredimethylester | 18 | 12 | 92 |

## Patentansprüche

1. Verfahren zur Herstellung von geradkettigen Acrylnitril-Dimeren**dadurch gekennzeichnet, dass** Acrylnitril in Gegenwart eines Rutheniumkatalysatorsund zusätzlich in Gegenwart eines Additivs dimerisiert wird, wobei das Additiv eine aromatische Hydroxyverbindung ist, die mindestens einen Alkyloxycarbonyl-Substituenten und mindestens einen weiteren Substituenten R am aromatischen Grundkörper trägt
oder
eine heteroaromatische Hydroxyverbindung ist, die mindestens einen Alkyloxycarbonyl-Substituenten und mindestens einen weiteren Substituenten R am heteroaromatischen Grundkörper trägt,
wobei R unabhängig voneinander
Halogen, Cyano, Amino, Amido, Urethan, Sulfonyl, Phosphonyl, Formyl, geradkettiges oder verzweigtes C₁-C₁₀-Alkyl, C₁-C₁₀-Alkoxy, C₁-C₁₀-Alkyloxo, C₁-C₁₀-Alkylsulfinyl, C₁-C₁₀-Alkylamino, C₁-C₁₀-Halogenalkyl oder C₁-C₁₀- Alkyloxycarbonyl bedeutet
und
die heteroaromatische Hydroxyverbindung als Heteroatom Stickstoff, Schwefel und/oder Sauerstoff enthält.

2. Verfahren gemäß Anspruch 1, **dadurch gekennzeichnet, dass** es sich bei dem Additiv um ein Phenolderivat handelt, wobei der Alkyloxycarbonyl-Substituent und/oder der Substituent R bezüglich der phenolischen Gruppe in ortho- und/oder para-Stellung stehen.

3. Verfahren gemäß einem oder mehreren der vorangegangenen Ansprüche 1 und 2, **dadurch gekennzeichnet, dass** es sich bei dem Additiv um 2-Hydroxy-1,3,5-benzoltricarbonsäuretrimethyl-ester, 2,4-Dihydroxy-3,6-dimethyl-benzoesäuremethylester, 5-Acetyl-2-hydroxy-benzoesäuremethylester oder 2-Hydroxy-1,5-benzoldicarbonsäuredimethylester handelt.

4. Verfahren gemäß einem oder mehreren der vorangegangenen Ansprüche 1 bis 3, **dadurch gekennzeichnet, dass** das Additiv in 0,001- bis 2-fachen molaren Mengen, bezogen auf die molare Menge an eingesetztem Acrylnitril, eingesetzt wird.

5. Verfahren gemäß einem oder mehreren der vorangegangenen Ansprüche 1 bis 4, **dadurch gekennzeichnet, dass** als Rutheniumkatalysatoren anorganische oder organische Rutheniumsalze oder Rutheniumkomplexe eingesetzt werden.

6. Verfahren gemäß Anspruch 5, **dadurch gekennzeichnet, dass** als Rutheniumkatalysator Dichloro-tetrakis-(dimethylsulfoxid)-ruthenium, Dichloro-tetrakis-(diphenylsulfoxid)-ruthenium, Dichloro-tetrakis-(diphenylsulfid)-ruthenium, Dibromo-tetrakis-(dimethylsulfoxid)-ruthenium, Dichloro-cyclooctadien-ruthenium, Dichloro-1,4-dicyanobutadien-ruthenium, Dichloro-bis-(acetylendicarbonsäure-methylester)-ruthenium, Ruthenium-bis-acetylacetonat, Dichloro-tris-(triphenoxy-phosphin)-ruthenium oder Dichloro-tetrakis-(dipropylsulfoxid)-ruthenium einge-setzt wird.

7. Verfahren gemäß einem oder mehreren der vorangegangenen Ansprüche 1 bis 6, **dadurch gekennzeichnet, dass** der Katalysator in einer Menge von 0,001 bis 5 mol%, bezogen auf die molare Menge an eingesetztem Acrylnitril, eingesetzt wird.

8. Verfahren gemäß einem oder mehreren der vorangegangenen Ansprüche 1 bis 7, **dadurch gekennzeichnet, dass** die Dimerisierung in einem Reaktionsmedium durchgeführt wird, welches mindestens eine Verbindung, wählbar aus Nitrilen, Sulfoxiden, Ether, Kohlenwasserstoffen, halogenierten Kohlenwasserstoffen, Amiden, Estern oder Wasser enthält.

9. Dichloro-tetrakis-(diphenylsulfoxid)-ruthenium.

10. Verwendung von Dichloro-tetrakis-(diphenylsulfoxid)-ruthenium als Katalysator.

11. Katalysatorsystem für die Dimerisierung von Acrylnitril, enthaltend einen Rutheniumkatalysator und ein Additiv gemäß einem oder mehreren der vorangegangenen Ansprüche 1 bis 3.

12. Verwendung von Additiven gemäß einem oder mehreren der vorangegangenen Ansprüche 1 bis 3 bei der Dimerisierung von Acrylnitril.

## Claims

1. Process for preparing straight-chain acrylonitrile dimer, **characterized in that** acrylonitrile is dimerized in the presence of a ruthenium catalyst and in the additional presence of an additive, the additive being an aromatic hydroxy compound bearing at least one alkyloxycarbonyl substituent and at least one further substituent R on the basic aromatic structure
or
a hetaromatic hydroxy compound bearing at least one alkyloxycarbonyl substituent and at least one further substituent R on the basic hetaromatic structure
where R is independently at each appearance
halogen, cyano, amino, amido, urethane, sulphonyl, phosphonyl, formyl, straight-chain or branched C₁-C₁₀-alkyl, C₁-C₁₀-alkoxy, C₁-C₁₀-alkyloxo, C₁-C₁₀-alkylsulphinyl, C₁-C₁₀-alkylamino, C₁-C₁₀-haloalkyl or C₁-C₁₀-alkyloxycarbonyl
and
the hetaromatic hydroxy compound contains nitrogen, sulphur and/or oxygen as heteroatom.

2. Process according to Claim 1, **characterized in that** the additive is a phenol derivative and the alkyloxycarbonyl substituent and/or the substituent R are disposed ortho and/or para relative to the phenolic group.

3. Process according to either or both of the preceding Claims 1 and 2, **characterized in that** the additive is trimethyl 2-hydroxy-1,3,5-benzenetricarboxylate, methyl 2,4-dihydroxy-3,6-dimethylbenzoate, methyl 5-acetyl-2-hydroxybenzoate or dimethyl 2-hydroxy-1,5-benzenedicarboxylate.

4. Process according to one or more of the preceding Claims 1 to 3, **characterized in that** the additive is used in 0.001 to 2 times the molar amount of acrylonitrile used.

5. Process according to one or more of the preceding Claims 1 to 4, **characterized in that** the ruthenium catalysts used are organic or inorganic ruthenium salts or ruthenium complexes.

6. Process according to Claim 5, **characterized in that** the ruthenium catalyst used is dichloro-tetrakis(dimethyl sulphoxide)-ruthenium, dichloro-tetrakis-(diphenyl sulphoxide)-ruthenium, dichloro-tetrakis-(diphenyl sulphide)-ruthenium, dibromo-tetrakis-(dimethyl sulphoxide)-ruthenium, dichloro-cyclooctadiene-ruthenium, dichloro-1,4-dicyanobutadiene-ruthenium, dichloro-bis-(methyl acetylenedicarboxylate)-ruthenium, ruthenium bis-acetylacetonate, dichloro-tris-(triphenoxy-phosphine)-ruthenium or dichloro-tetrakis-(dipropyl sulphoxide)-ruthenium.

7. Process according to one or more of the preceding Claims 1 to 6, **characterized in that** the catalyst is used in an amount of 0.001 to 5 mol%, based on the molar amount of acrylonitrile used.

8. Process according to one or more of the preceding Claims 1 to 7, **characterized in that** the dimerization is carried out in a reaction medium which includes at least one compound selectable from nitriles, sulphoxides, ethers, hydrocarbons, halogenated hydrocarbons, amides, esters or water.

9. Dichloro-tetrakis-(diphenyl sulphoxide)-ruthenium.

10. Use of dichloro-tetrakis-(diphenyl sulphoxide)-ruthenium as catalyst.

11. Catalyst system for dimerization of acrylonitrile, comprising a ruthenium catalyst and an additive according to one or more of the preceding Claims 1 to 3.

12. Use of additives according to one or more of the preceding Claims 1 to 3 in the dimerization of acrylonitrile.

## Revendications

1. Procédé pour la préparation de dimères d'acrylonitrile linéaires, **caractérisé en ce qu'**on dimérise de l'acrylonitrile en présence d'un catalyseur à base de ruthénium et en outre en présence d'un additif, l'additif étant un composé hydroxy aromatique qui porte au moins un substituant alkyloxycarbonyle et au moins un autre substituant R sur le corps de base aromatique
ou
un composé hydroxy hétéroaromatique qui porte au moins un substituant alkyloxycarbonyle et au moins un substituant R sur le corps de base hétéroaromatique,
R signifiant, indépendamment l'un de l'autre,
halogène, cyano, amino, amido, uréthane, sulfonyle, phosphonyle, formyle, C₁-C₁₀-alkyle, C₁-C₁₀-alcoxy, C₁-C₁₀-alkyloxo, C₁-C₁₀-alkylsulfinyle, C₁-C₁₀-alkylamino, C₁-C₁₀-halogénoalkyle ou C₁-C₁₀-alkyloxycarbonyle linéaire ou ramifié
et
le composé hydroxy hétéroaromatique contenant comme hétéroatome de l'azote, du soufre et/ou de l'oxygène.

2. Procédé selon la revendication 1, **caractérisé ce qu'**il s'agit, pour l'additif, d'un dérivé du phénol, le substituant alkyloxycarbonyle et/ou le substituant R se trouvant en position ortho et/ou para par rapport au groupe phénolique.

3. Procédé selon l'une ou plusieurs des revendications précédentes 1 et 2, **caractérisé en ce qu'**il s'agit, pour l'additif, de l'ester triméthylique de l'acide 2-hydroxy-1,3,5-benzènetricarboxylique, de l'ester méthylique de l'acide 2,4-dihydroxy-3,6-diméthyl-benzoïque, de l'ester méthylique de l'acide 5-acétyl-2-hydroxy-benzoïque ou de l'ester diméthylique de l'acide 2-hydroxy-1,5-benzènedicarboxylique.

4. Procédé selon l'une ou plusieurs des revendications précédentes 1 à 3, **caractérisé en ce que** l'additif est utilisé en des quantités molaires représentant 0,001 à 2 fois la quantité molaire d'acrylonitrile utilisée.

5. Procédé selon l'une ou plusieurs des revendications précédentes 1 à 4, **caractérisé en ce qu'**on utilise comme catalyseurs à base de ruthénium des sels ou des complexes de ruthénium inorganiques ou organiques.

6. Procédé selon la revendication 5, **caractérisé en ce qu'**on utilise comme catalyseur à base de ruthénium, du dichloro-tétrakis-(diméthylsulfoxyde)-ruthénium, du dichloro-tétrakis-(diphénylsulfoxyde)-ruthénium, du dichloro-tétrakis-(diphénylsulfure)-ruthénium, du dibromo-tétrakis-(diméthylsulfoxyde)-ruthénium, du dichloro-cyclooctadiène-ruthénium, du dichloro-1,4-dicyanobutadiène-ruthénium, du dichloro-bis-(ester méthylique de l'acide acétylènedicarboxylique)-ruthénium, du bis-acétylacétonate de ruthénium, du dichloro-tris-(triphénoxyphosphine)-ruthénium ou du dichloro-tétrakis-(dipropylsulfoxyde)-ruthénium.

7. Procédé selon l'une ou plusieurs des revendications précédentes 1 à 6, **caractérisé en ce que** le catalyseur est utilisé en une quantité de 0,001 à 5% en mole par rapport à la quantité molaire d'acrylonitrile utilisée.

8. Procédé selon l'une ou plusieurs des revendications précédentes 1 à 7, **caractérisé en ce que** la dimérisation est réalisée dans un mélange réactionnel qui contient au moins un composé pouvant être choisi parmi les nitriles, les sulfoxydes, les éthers, les hydrocarbures, les hydrocarbures halogénés, les amides, les esters ou l'eau.

9. Dichloro-tétrakis-(diphénylsulfoxyde)-ruthénium.

10. Utilisation de dichloro-tétrakis-(diphénylsulfoxyde)-ruthénium comme catalyseur.

11. Système de catalyseur pour la dimérisation d'acrylonitrile, contenant un catalyseur à base de ruthénium et un additif selon l'une ou plusieurs des revendications précédentes 1 à 3.

12. Utilisation d'additifs selon l'une ou plusieurs des revendications précédentes 1 à 3 lors de la dimérisation de l'acrylonitrile.
